# EUROPEAN PATENT APPLICATION

(11) **EP 4 364 785 A1**
(43) Date of publication of application: **08.05.2024**
(21) Application number: 23206741.3
(22) Date of filing: 30.10.2023
(51) Int. Cl.: A61M 39/18, A61J 1/20, A61M 39/20

(54) **DOUBLE-LOCK STERILE ENTRY INTRAVENOUS PORT AND SYRINGE INTERFACE SYSTEM**

(30) Priority: 04.11.2022 US 202217981366
(71) Applicant: DoubleLock Healthcare, Inc., Palm Springs California 92264 (US)
(72) Inventor: DEPTALA, Arthur, Palm Springs, CA 92264 (US); COCHRAN, Jonas, Palm Springs, CA 92264 (US)
(74) Representative: Derry, Paul Stefan

(57) **Abstract**

A sterile interface system for placement between a syringe and an IV port to provide a closed system for drawing up and injecting liquid drugs, comprises an IV cannula interface for connection to an IV delivery tube, a syringe interface for a connection to a drug delivery syringe and a shrouded interface positioned between the IV cannula interface and the syringe interface. The sterile interface system includes several puncturable silicone structures which are pierced by a cannula mounted longitudinally within the IV cannula interface to provide a sterile flow path.

## Description

Described and shown herein is a double lock sterile entry IV port and syringe interface system comprising a closed system that decreases or prevents patient exposure to bacteria when fluids are drawn up and injected in the field, at the bedside, or in the operating room.

### BACKGROUND

The E. Wong US Patent 9,352,141 is directed to a system comprising a sterile entry IV port and a sterile entry syringe delivery mechanism. In US Patent 9,352,141 the IV port and syringe delivery mechanism can each have a cover over their respective entry or exit point. These covers keep the components of the IV port and/or syringe delivery mechanism sterile. The IV port and/or syringe delivery mechanism covers can each include a mechanism, such as a push mechanism, placed adjacent to the entry or exit point. When sufficient force is applied to this mechanism, the cover is raised away from the entry or exit point, allowing an IV port and syringe delivery mechanism to be joined using, for example, a single or double Luer Lock^{™} system. By enabling the cover to be raised without touching the entry or exit points, embodiments shown therein can prevent contamination of the IV port and syringe delivery mechanism. Further, the covers and/or the system comprising the covers can be reusable.

Prior clinical practice generally provides for the delivery of sterile solutions (e.g., drugs, fluids, and/or blood) by attaching uncapped Luer Lock^{™} syringes to uncovered Luer Lock^{™} needleless IV ports. In order to ensure a sterile environment, practitioners currently either swab the needleless IV port with alcohol or use single-use alcohol impregnated caps that are placed on the needleless IV port for about three minutes before delivery. This practice was found to not be adequate as patients continued to acquire healthcare-associated infections (HAI) because these prior practices were not always effective in ensuring a sterile environment.

Various other systems have been designed to aid medical professionals in the intravenous delivery of sterile solutions. Such systems are described, for example, in U.S. Pat. Nos. 6,003,556 to Brugger et al.; 5,881,774 to Utterberg; and 5,817,067 to Tsukada; as well as U.S. Pat. Pub. Nos. 2012/0238965 and 2010/0298783 to Chang. The figures and descriptions of U.S. Pat. No. 9,352,141 and all five of these patents and publications are incorporated herein in their entirety by reference. However, these systems were lacking in many regards. For instance, none of these systems ensures a sterile environment, and extra precautions such as those described above must therefore accompany their use.

Applicant's prior device shown and claimed in US 9,352,141 addressed many of the prior potential contamination issues. The system shown and described therein is directed to a single or double-lock sterile entry intravenous port and syringe system. The system includes caps which can be hinged. These caps cover the entry points of the IV port and/or syringe and are intended to prevent the contamination of these areas. When ready for use a portion of each cap can be removed from the port by pressing on a mechanism spaced sufficiently away to prevent contamination by finger contact with the port opening. In turn, pressing on the mechanism causes the cap cover to raise, allowing access to the entry point of the IV port and/or syringe delivery mechanism. However, while that device was an improvement over prior devices it was still subject to bacterial contamination and the transmission of hospital-acquired infections.

### SUMMARY

The Double Lock Sterile Entry IV Port and Syringe Interface System disclosed herein is a closed system that further decreases or prevents patient exposure to bacteria when fluids are drawn up and injected in the field, at the bedside, or in the operating room. Along with potentially reducing or preventing HAIs, an additional application of this system is as a transfer device that decreases drug vapors and leakage in the administration of hazardous drugs such as chemotherapy. Throughout medical history there has been a continual effort to decrease or prevent the incidence of HAIs. As the science and technology of medical devices has improved, new medical devices have come onto the market that eliminate areas of potential exposure to bacterial contamination. This novel medical device is a next generation evolution in IV port and syringe connectors to prevent contamination of the fluids delivered or collected.

The improved sterile field contamination protection provided to combinations of an IV port and syringe, such as shown in US Patent 9,352,141, incorporated herein in its entirety by reference, and other prior devices, comprises a shrouded interface including food-grade silicone diaphragms provided as a covering along the flow path of fluids between the IV port and syringe. Benefits of the food-grade silicone diaphragm include high resistant to damage and degradation from extreme temperatures, it does not harden, crack, peel, crumble, dry out, rot or become brittle over time, it is lightweight, saves space, is easy to transport, is made from an abundant natural resource which is non-toxic and odorless, does not contain BPA, latex, lead, or phthalates, and it is considered to be a non-hazardous waste. Due to its resiliency, non-porous surface and sustainability, food grade silicone is also considered to provide a superior bacterial-free interface. Still further, the food-grade silicone has inherent antimicrobial properties in regard to both gram-positive and gram-negative microbial strains and fungi.

### BRIEF DESCRIPTION OF THE DRAWINGS

Figure 1 is a side perspective view of the assembled sterile entry IV port and syringe interface system incorporating features of the invention attached to a fluid delivery syringe.
Figure 2 is a longitudinal cross section side view of the system of Figure 1 prior to attachment of the IV cannula interface to the assembled shrouded interface and syringe interface, the IV cannula interface, shrouded interface, and syringe interface cutaway to show the internal components.
Figure 3 is an expanded side view showing the three main components of the sterile entry IV port and syringe interface system with the syringe interface attached to a standard drug delivery syringe.
Figure 4 is a side view of the sterile entry IV port and syringe interface system of Fig. 3, wherein the sterile entry IV port is partially inserted into the shrouded interface, the syringe interface cutaway to show the internal components.
Figure 5 is a longitudinal cutaway side view of the fully assembled sterile entry IV port and syringe interface system of Fig. 3.
Figure 6 is a side view of the shrouded interface showing the internal spring and four leaflets on the second silicone disc.
Figure 7 is a perspective side view of the IV cannula interface.
Figure 8 is a perspective side view of the syringe interface showing the notches and channels of the locking mechanism in the syringe interface.
Figure 9 is a perspective top view of an embodiment of the IV cannula interface with an attached IV bottle spike used in the assembly procedure of the syringe interface system of Figure 1.

### DETAILED DESCRIPTION

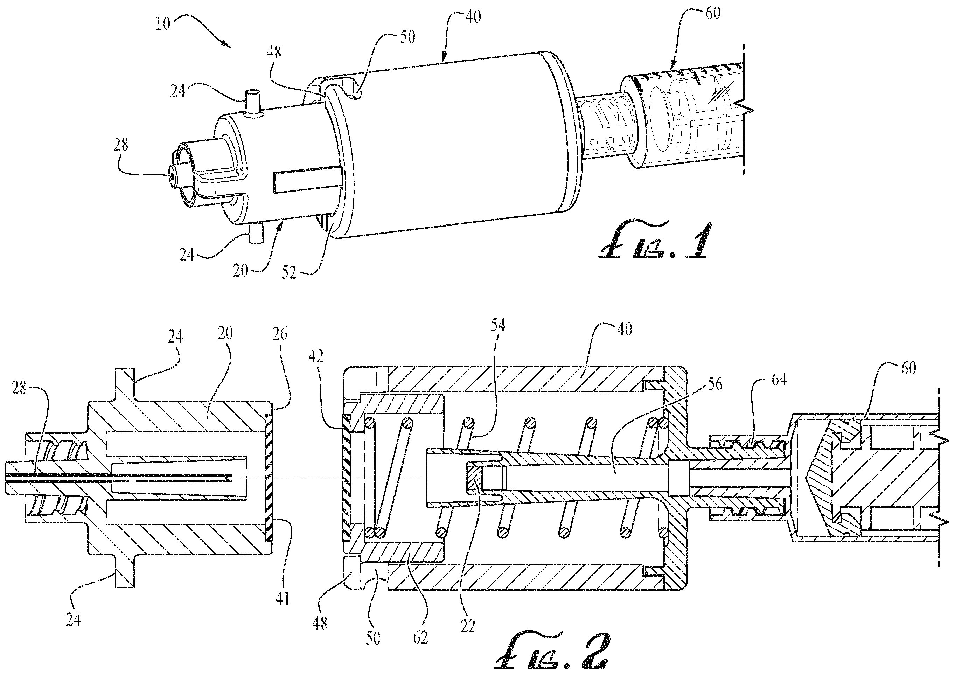

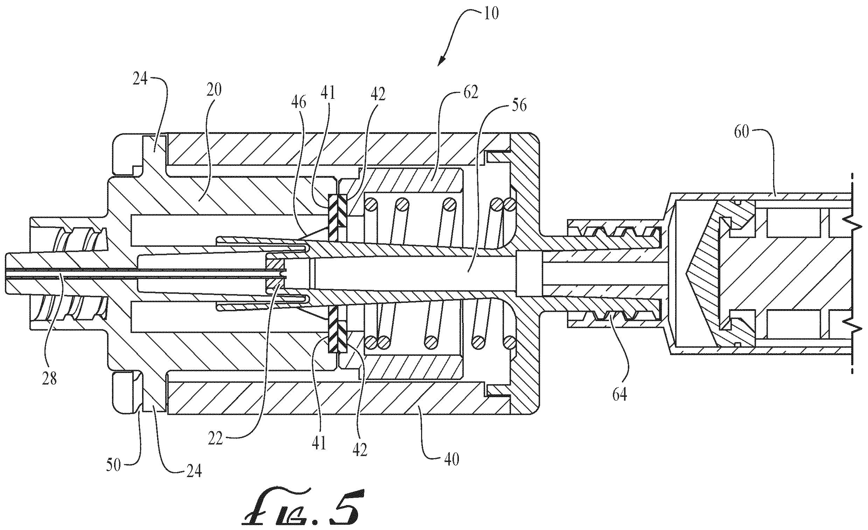

Shown and described herein is an improved Double Lock Sterile Entry IV Port and Syringe System 10, also referred to as a three-part interface, for positioning between a syringe 60 and an IV port (not shown) to provide a closed system for drawing up and injecting liquid drugs, shown in its assembled form in Figure 1. With reference to Figures 1-8, and particularly Figures 2-5 which show cutaway views of the various sub-assemblies, the three-part interface 10 comprises:
a) an IV cannula interface 20 which has a bung piercing cannula 28 mounted therein and a first silicone disc 41 covering an outer surface 26 of the cannula interface 20,
b) a syringe interface 40 with a cannula receiving extension 56 extending internally from one end thereof and a self-sealing, pierceable bung 22 mounted therein and
c) a shrouded interface 62 with a second silicone disc 42 inserted in and covering the other one end of the syringe interface 40 to provide a seal on the outer end, both the bung 22 and the silicone discs 41, 42 preferably formed from food-grade silicone film.
The syringe interface 40 has a first end configured to receive the shrouded interface 62 and a second end to receive the cannula receiving extension 56. A threaded end 64 of the cannula receiving extension 56 extends beyond the syringe interface 40 to provide a leak proof attachment to a standard drug delivery syringe 60.

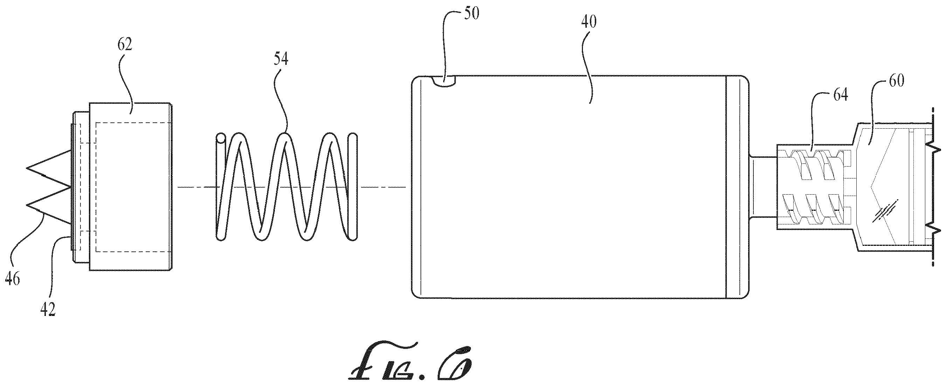

With reference to Figure 6, the shrouded interface 62 comprises a second silicone disc 42 with four slits 44 therein to form four leaflets 46, the four leaflets 46 creating a sealed diaphragm in its closed configuration. Once the IV cannula interface 20, the syringe interface 40 and shrouded interface 62 are assembled, a forward end of the cannula receiving extension 56 pierces the second silicone disk 42 causing the leaflets 46 to be displaced forward into the IV cannula interface 40. Following piercing of the bung 22 by the bung-penetrating cannula 28 a flow channel is formed completing a sterile fluid flow connection from the syringe 60 through the system 10 to the IV Port (not shown). The actual fluid connections are thus protected from exposure to pathogens creating a continuous sterile chamber between the IV port and the syringe 60.

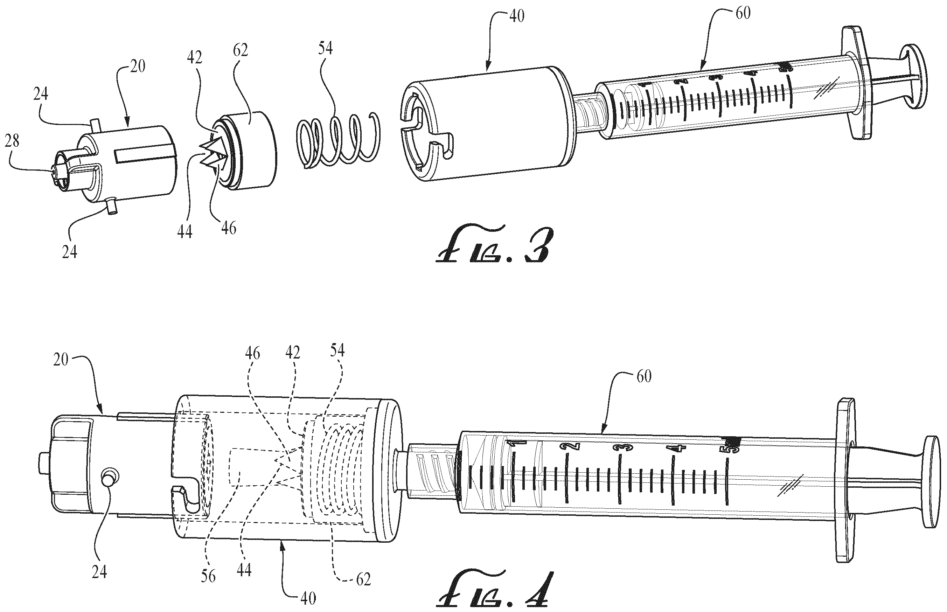

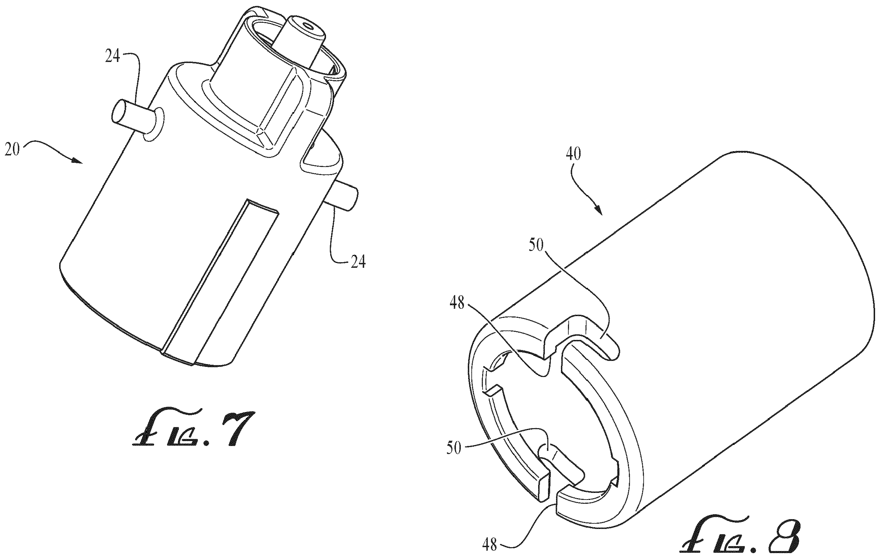

In use, an end of the IV cannula interface 20, best shown in Figure 7 is connected to the shrouded interface 62 and syringe interface 40 by pushing the parts together and twisting the IV cannula interface 20 such that tabs 24 extending from the outer surface thereof slide into a notches 48 and connected channels 50 on the distal end 52 of the syringe interface 40 (see Figures 4 and 8) to form a sliding lock, said movement also compressing spring 54 within the shrouded interface 62 when positioned within the syringe interface 40. Twisting the cannula interface 20 causes the slide lock to travel along the channels 50 in the syringe interface 40. The locking mechanism on the IV port and syringe also engage the IV cannula interface 20 and the syringe interface 40 to secure the connection. Connecting the parts causes the cannula receiving extension 56 in the syringe interface 40 to press the tabs 24 forward into the IV cannula interface 20 in a position to receive therein the bung-penetrating cannula 28 that has now pierced the bung 22. The slide lock is used to guide the IV cannula interface 20 into the shrouded interface 62 on/into the syringe interface 40. Once the sterile bung-penetrating cannula 28 penetrates the silicone bung 22 fluids are allowed to flow between the syringe and the IV port through the sterile chamber created by the Double Lock Sterile Entry IV Port and Syringe System 10, (the three-part interface).

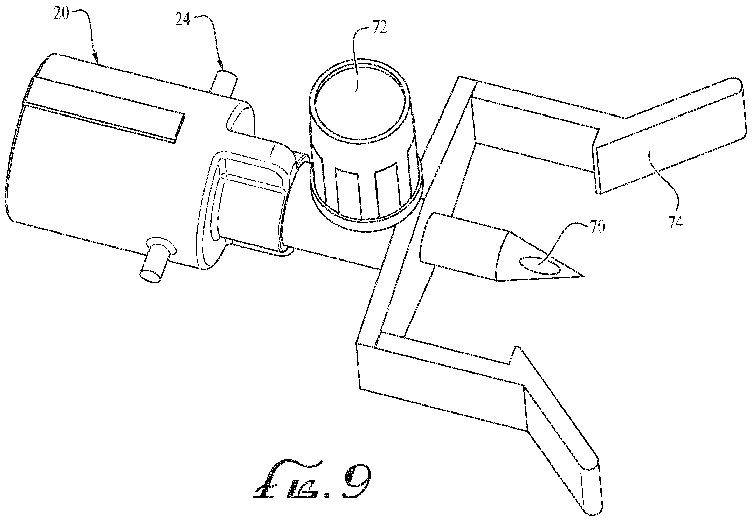

Figure 9 shows an embodiment of the IV cannula interface with an attached IV bottle spike used in the assembly procedure of the syringe interface system of Figure 1.

In a preferred embodiment the double lock sterile entry IV port and syringe system 10 described above and shown in Figures 1-8 is provided to the user as three separate components, namely the IV cannula interface 20, the shrouded interface 62, and the syringe interface 40, each in a separate sterile pouch (not shown). A fourth component, shown in Figure 9, used only in the assembly procedure, also provided in a sterile pouch, comprises an IV cannula interface 20 which has attached thereto a fluid control valve 72 and an IV spike 70 and wings 74 for receiving and grasping the top of an IV bottle (not shown) the IV spike 70 provide for piercing a seal on said IV bottle.

To assemble the double lock sterile entry IV port and syringe System 10 the user assembly
a) The IV cannula interface 20 with IV spike 70 is attached to a vial of sterile saline.
b) The shrouded interface is inserted in to the syringe interface as shown in Figure 2 and the IV cannula interface 20 with IV spike 70 (with attached vial of sterile saline) is connected to the shrouded interface 62. This causes the cannula 28 to pierce the first and second silicone discs 41, 42 and the bung 22.
c) The plunger in the syringe 60 is withdrawn pulling the sterile saline through the system 10.
d) The IV cannula interface 20 with IV spike 70 and vial of sterile saline is removed, replaced by the sterile IV cannula interface 20 (see Figure 1) and the completely assembled double lock sterile entry IV port and syringe System 10 filled with sterile saline is then attached to and IV port for drug delivery by standard procedures.

At the conclusion of the transfer of the drug from the syringe to the IV port, the syringe and IV port are disconnected by a quick twist in the opposite direction. Pulling the IV port and syringe apart initiates the spring in the syringe interface to assist in the separation. The bung-penetrating cannula is then retracted, the silicone bung seal reseals, and the silicone diaphragm leaflets return to their original closed position protecting the sterile chamber so that the IV port and syringe can be disconnected and reconnected in a reusable fashion.

The present invention provides embodiments of double lock sterile entry IV port and syringe interface system for placement between a sterile entry IV port and a sterile entry syringe delivery mechanism that can be joined together as part of a fluid delivery system. Embodiments of the sterile entry IV ports and sterile entry syringe delivery mechanisms can include covers over the entry or exit points of the IV port and the syringe delivery mechanism such that the entry/exit point and all of the components behind the entry/exit point remain sterile. The movement of the covers can be caused by the application of force to a portion of the cover structure at a point spaced from the entry/exit point in order to avoid contamination of the entry/exit point. Thus, the necessity of swabbing or otherwise sterilizing the entry/exit point can be eliminated. In some embodiments the cover and movement system are reusable.

The silicone bung 22 and adjacent components create a tortuous flow path to minimize/prevent transfer of potential pathogens. The silicone bung is solid and provides a vacuum-tight seal along the fluid path. The silicone bung once penetrated by the needle/cannula allows fluid or drug to pass through the interface assembly. The silicone bung reseals (is self-sealing); as the needle is withdrawn the bung wipes the needle minimizing exposure to drug vapors and fluid leakage.

It is understood that when an element is referred to as being "on" another element, it can be directly on the other element or intervening elements may also be present. Furthermore, relative terms such as "inner", "outer", "upper", "above", "lower", "beneath", and "below", and similar terms, may be used herein to describe a relationship of one element to another. Terms such as "higher", "lower", "wider", "narrower", and similar terms, may be used herein to describe relative relationships. It is understood that these terms are intended to encompass different locations and orientations in addition to the orientation depicted in the figures.

Although the terms a, first, second, etc., may be used herein to describe various elements, components, regions and/or sections, these elements, components, regions, and/or sections should not be limited by these terms. These terms are only used to distinguish one element, component, region, or section from another. Thus, unless expressly stated otherwise, a first element, component, region, or section discussed below could be termed a second element, component, region, or section without departing from the teachings of the present invention.

Embodiments of the invention are described herein with reference to illustrations that are schematic illustrations. As such, the actual dimensions of elements can be different, and variations from the shapes of the illustrations as a result, for example, of manufacturing techniques and/or tolerances are expected. Thus, the elements illustrated in the figures are schematic in nature and their shapes are not intended to illustrate the precise shape of the product and are not intended to limit the scope of the invention.

Although the present invention has been described in detail with reference to certain preferred configurations, assembly and use thereof, other versions or combinations of the disclosed embodiments are possible. Also, while the present invention is shown and describe as three sub-assemblies the product as delivered to the users can include one or more of the subassemblies assembled together or all of the subassemblies can be provide partially assembled or as a single interface unit for attachment to the Syringe and IV port. Therefore, the spirit and scope of the invention should not be limited to the versions described above or shown in the Figures.

## Claims

1. A sterile interface system for placement between a syringe and an IV port to provide a closed system for drawing up and injecting liquid drugs, comprising:
a) an IV cannula interface,
b) a syringe interface, and
c) a shrouded interface between the IV cannula interface and the syringe interface.

2. The sterile interface system of claim 1 wherein said IV cannula interface has a first end configured to receive an IV tube connection, a bung piercing cannula mounted longitudinally within the IV cannula interface and extending from the first end toward a second end of the IV cannula interface, a first pierceable silicone disc covering the second end of said the IV cannula interface, the IV cannula interface having structure on the outer surface thereof for interacting with and connecting the IV cannula interface to a second end of the syringe interface.

3. The sterile interface system of claim 1 wherein the syringe interface includes a cannula receiving extension extending internally and longitudinally from a first end thereof and a self-sealing, pierceable bung mounted therein, said bung configured to receive the bung piercing cannula within the IV cannula interface, the syringe interface configured to receive the shrouded interface positioned in the second end of the syringe interface, the syringe interface further including a spring therein compressible upon insertion of the IV cannula interface into the second end of the syringe interface and through the shrouded interface.

4. The sterile interface system of claim 3 wherein the shrouded interface comprises a structure located within the second end of the syringe interface, the second end of the syringe interface further configured to receive the second end of the IV cannula interface in a connecting manner, the shrouded interface configured to receive the bung piercing cannula therethrough.

5. The sterile interface system of claim 4 wherein connection of the IV cannula interface in a locking manner to the syringe interface causes the shrouded interface previously placed in the syringe interface to slide within the syringe interface, compressing said spring, the bung piercing cannula mounted within the cannula interface extending into the cannula receiving extension and to pierce the bung.

6. The sterile interface system of claim 3 wherein a second silicone disc covers a first end of the shrouded interface, the second silicone disc pierced by the bung piercing cannula within the IV cannula interface and the cannula receiving extension as the IV cannula interface is connected to the syringe interface.

7. The sterile interface system of claim 3 wherein the bung and the silicone discs are formed from a food-grade silicone material.
